# EUROPÄISCHE PATENTANMELDUNG

(11) **EP 4 806 408 A1**
(43) Veröffentlichungstag der Anmeldung: **16.09.2026**
(21) Anmeldenummer: 25209483.4
(22) Anmeldetag: 17.10.2025
(51) Int. Cl.: A61F 13/15, A44B 18/00, A61F 13/62, B29C 65/08, B29C 65/00

(54) **MODULARER AMBOSS FÜR EINE ULTRASCHALLSCHWEISSANLAGE, BAUSATZ FÜR EINEN MODULAREN AMBOSS UND RAPID-PROTOTYPING-VERFAHREN MIT EINEM MODULAREN AMBOSS**

(30) Priorität: 11.03.2025 DE 102025109236; 11.03.2025 DE 202025101295 U; 28.04.2025 EP 25173024
(71) Anmelder: Gottlieb Binder GmbH & Co. KG, 71088 Holzgerlingen (DE)
(72) Erfinder: HEEPE, Lars, 72072 Tübingen (DE); CARTER, Nick, 65191 Wiesbaden (DE)
(74) Vertreter: Kohler Schmid Möbus Patentanwälte

(57) **Zusammenfassung**

Die Erfindung betrifft einen modularen Amboss (10) für eine Ultraschallschweißanlage (30), welcher insbesondere aus einem Bausatz aus einer Mehrzahl von Ambosssegmenten (100) und ggf. weiteren Elementen (200) konfiguriert werden kann. Ambosssegmente (100) dienen im Zusammenwirken mit einer Sonotrode (20) zur Erzeugung von Schweißstellen (42) bzw. Schweißlinien (42'). Als weitere Elemente (200) kommen beispielsweise Distanzelemente (201), Führungselemente (202), Prägeelemente (203) oder Schneidelemente (204) in Frage. Ambosssegmente (100) und ggf. weitere Elementen (200) sind lösbar aneinander befestigt, sodass der Amboss (10) mehrfach neu konfiguriert werden kann. Die Erfindung betrifft auch ein Verfahren (300), wobei ein erfindungsgemäßer Amboss (10) in wenigstens zwei Konfigurationen, oder anders ausgedrückt wenigstens zwei erfindungsgemäße Ambosse (10), die aus demselben Bausatz erhalten sein können, für Probeverschweißungen genutzt wird/werden. Auf Basis der Probeverschweißungen wird anschließend eine vorteilhafte Konfiguration identifiziert und ein entsprechender nichtmodularer (d. h. weniger oder nicht segmentierter) Amboss auf Basis einer der zuvor verwendeten Konfigurationen des modularen Ambosses (10) hergestellt und für Serienverschweißungen genutzt.

## Beschreibung

Die Erfindung betrifft einen Amboss für eine Ultraschallschweißanlage. Die Erfindung betrifft gleichermaßen eine Ultraschallschweißanlage mit einem Amboss. Die erfindungsgemäße Ultraschallschweißanlage kann besonders bevorzugt zum Verschweißen eines Haftverschlussteils, das vorstehende Verschlusselemente aufweist, und eines Festlegteils verwendet werden. Die Erfindung betrifft ferner ein Verfahren zum Festlegen der Gestaltung eines Ambosses für eine Ultraschallschweißanlage.

An Ultraschallschweißanlagen können Werkstücke, insbesondere Werkstücke, welche thermoplastische Kunststoffe aufweisen, im Rahmen eines Ultraschallschweißvorgangs miteinander verbunden werden. Dabei werden hochfrequente mechanische Schwingungen genutzt, um das Material der Werkstücke durch über Reibung erzeugte Wärme lokal aufzuschmelzen bzw. zu plastifizieren und miteinander zu verbinden. Die mechanischen Schwingungen werden in der Regel mittels eines ersten Schweißwerkzeugs in Form einer Sonotrode auf die zu verschweißenden Materialien übertragen. Typischerweise liegt die Sonotrode dabei an einem der Werkstücke an. Ein zweites Schweißwerkzeug in Form eines Ambosses befindet sich gegenüber der Sonotrode. Der Amboss dient als Auflage bzw. Gegenlager für die Sonotrode bzw. die Werkstücke. Typischerweise erfolgt ein Verschweißen somit in einem Schweißspalt zwischen der Sonotrode und dem Amboss. Der Amboss kann insbesondere walzenförmig rotierend ausgebildet sein, um ein Verschweißen in einem kontinuierlichen Prozess zu begünstigen. Ein Ultraschallschweißverfahren kann zur vergleichsweise schnellen, energieeffizienten und präzisen Verbindung der Werkstücke genutzt werden.

Werkstücke können beispielsweise ein Haftverschlussteil mit vorstehenden Verschlusselementen und ein Festlegeteil, insbesondere aus Vlies, sein. Durch ein Verschweißen derselben kann ein vorteilhaftes Haftverschlusssystem hergestellt werden, welches sich beispielsweise zur Anordnung an einem Hygieneartikel, zum Beispiel einer Windel, eignet. EP 3 408 067 B1 und EP 2 815 733 A1 beschreiben jeweils Verfahren zum Herstellen eines Haftverschlusssystems, wobei ein Verschlusselemente aufweisendes Haftverschlussteil mittels eines Ultraschallschweißverfahrens mit einem Festlegeteil verschweißt wird.

Zur Beeinflussung der Geometrie der erzeugten Schweißstellen sowie insbesondere weiterer Eigenschaften, wie beispielsweise der "Tiefe" der Schweißstellen, kann der Amboss entsprechend ausgestaltet werden. Insbesondere kann der Amboss diskrete Schweißvorsprünge aufweisen, deren Form und Ausdehnung für den Erhalt der gewünschten Schweißstellen entsprechend angepasst ist.

Die Herstellung eines jeweiligen Ambosses, insbesondere wenn dieser komplexe, ggf. verschiedenartige, Schweißvorsprünge aufweisen soll, kann äußerst zeitaufwändig und kostenintensiv sein. Bei der Herstellung von Probeverschweißungen bzw. kurzfristigen Anpassungen bei Kleinserienproduktionen wirkt sich dies ungünstig aus. Darüber hinaus kann es wünschenswert sein den Amboss in flexibler Weise mit weiteren Strukturen auszustatten, welche einen Produktionsprozess unterstützen können, beispielsweise zur Erzeugung von Schnitten, Einprägungen oder zur Führung der Werkstücke.

### Aufgabe der Erfindung

Es ist eine Aufgabe der Erfindung, es zu erleichtern, Ultraschallschweißverfahren produktspezifisch auszugestalten. Es ist auch eine Aufgabe der Erfindung, ein rationelles Verfahren für die Festlegung der Gestaltung eines Ambosses für eine Ultraschallschweißanlage zu schaffen.

### Beschreibung der Erfindung

Diese Aufgabe wird erfindungsgemäß gelöst durch einen modularen Amboss gemäß Patentanspruch 1, eine Ultraschallschweißanlage gemäß Patentanspruch 15, die Verwendung einer Ultraschallschweißanlage gemäß Patentanspruch 16, einen Bausatz für einen modularen Amboss gemäß Patentanspruch 17 sowie ein Verfahren gemäß Patentanspruch 18.

### Erfindungsgemäßer Amboss

Die Aufgabe wird mithin gelöst durch einen modularen Amboss für eine Ultraschallschweißanlage, aufweisend eine Mehrzahl von Ambosssegmenten zum Zusammenwirken mit einer Sonotrode, wobei die Ambosssegmente lösbar aneinander befestigt sind.

Der Amboss weist zumindest zwei Ambosssegmente auf. Vorzugsweise kann er mehr als zwei Ambosssegmente aufweisen. Die Ambosssegmente müssen im vorliegenden Kontext nicht unmittelbar aneinander befestigt sein. Typischerweise befindet sich zwischen zwei Ambosssegmenten zumindest ein Distanzelement.

Durch Auswahl geeigneter Ambosssegmente und ggf. die Wahl der Ausrichtung der Ambosssegmente relativ zueinander kann der Amboss einfach und schnell an eine jeweilige Schweißaufgabe angepasst werden. Der Amboss kann je nach Anwendungsfall in geeigneter Weise konfiguriert werden, um die gewünschten Eigenschaften der Schweißverbindung zu erhalten. Entsprechend können Eigenschaften der Schweißverbindung, insbesondere deren Geometrie, durch die Auswahl und/oder Anordnung der verwendeten Ambosssegmente beeinflusst werden. Ferner kann der Amboss je nach Bedarf zum Verschweißen verschiedenartiger Werkstücke konfiguriert werden.

Das Lösen bzw. Befestigen der Ambosssegmente kann insbesondere unter Werkzeugeinsatz erfolgen. Alternativ kann ein manuell lösbarer Schnellverschluss vorgesehen werden. Zur Befestigung können ggf. Formschlusselemente vorgesehen sein, insbesondere zur Fixierung einer Drehstellung der Ambosssegmente.

Vorzugsweise weist der Amboss eine Drehachse auf.

Der Amboss ist bevorzugt walzenförmig ausgebildet. Durch ein Abrollen des Ambosses relativ zu den zu verschweißenden Werkstücken können somit hohe Prozessgeschwindigkeiten bei Verschweißen erreicht werden.

Die Drehachse ist insbesondere als mechanische Welle ausgebildet, welche sich beim Abrollen des Ambosses mit den Ambosssegmenten dreht. Demnach können die Ambosssegmente vorzugsweise so auf der Drehachse angeordnet sein, dass sie sich nicht relativ zu dieser verdrehen lassen.

Die Drehachse durchgreift vorzugsweise eine zentrale Ausnehmung der Ambosssegmente.

Die Ambosssegmente sind typischerweise scheibenförmig. Insbesondere können sie mittels der zentralen Durchgangsausnehmung auf die Drehachse aufgeschoben werden. Ein Sichern gegenüber einem Verdrehen der Ambosssegmente auf der Drehachse kann beispielsweise über einen Formschluss zwischen der Drehachse und einem entsprechend ausgebildeten Rand der zentralen Ausnehmung erfolgen. Alternativ oder zusätzlich kann zwischen der zentralen Ausnehmung und der Drehachse ein Presssitz ausgebildet sein.

Vorzugsweise können die Ambosssegmente und, sofern vorhanden, zwischen und/oder neben den Ambosssegmenten angeordnete weitere Elemente, in axialer Richtung auf der Drehachse fixiert sein, insbesondere durch zumindest einen lösbaren Kragen.

Bevorzugt weist der Amboss zur Fixierung der Ambosssegmente und ggf. weiterer Elemente in Axialrichtung zwei Krägen auf, von denen zumindest einer lösbar ist. Der lösbare Kragen selbst kann mittels bekannter Methoden auf der Drehachse fixiert werden (beispielsweise mithilfe von Stiftschrauben). Insbesondere kann der Kragen Sicherungselemente aufweisen, welche ein unbeabsichtigtes Lösen des Kragens bei der Benutzung des Ambosses verhindern.

Vorzugsweise trägt der Kragen, bzw. tragen die Krägen zum Verspannen der Ambosssegmente und ggf. der weiteren Elemente bei. Insbesondere können mithilfe des Kragens/der Krägen die Ambosssegmente und ggf. weiteren Elemente in axialer Richtung zusammengedrückt werden.

Vorzugsweise ist die Drehachse beidseits drehbar in einem Lagerblock gelagert.

Über den jeweiligen Lagerblock kann der Amboss an weitere Strukturen der Ultraschallschweißanlage angebunden werden bzw. grundsätzlich abgestützt werden. Vorzugsweise kann die Drehachse zur Neukonfiguration des Ambosses bzw. zu dessen Austausch einfach aus ihrer "Aufhängung" zwischen zwei Lagerblöcken entfernt werden. Alternativ oder zusätzlich können die Lagerblöcke mitsamt der Drehachse von der Ultraschallschweißanlage entfernt werden. Auch sind Ausführungsformen denkbar, wobei eine Drehachse ggf. fest an der Ultraschallschweißanlage verbaut ist und wobei Ambosssegmente und ggf. weitere Elemente mehrteilig ausgebildet sind und um die Drehachse herum gelegt und fixiert werden können.

Wie oben erwähnt, weist der Amboss vorzugsweise wenigstens ein Distanzelement auf, insbesondere wobei das Distanzelement zwischen zwei Ambosssegmenten angeordnet ist.

Gleichermaßen kann ein Distanzelement zwischen weiteren Elementen oder zwischen einem Ambosssegment und einem weiteren Element oder zu einem der Krägen hin angeordnet sein. Insbesondere kann ein Amboss mehrere Distanzelemente aufweisen. Distanzelemente eines Ambosses können unterschiedliche Ausdehnungen, insbesondere in axialer Richtung, aufweisen. Alternativ oder zusätzlich können Distanzelemente (zur Vergrößerung der Distanz) unmittelbar nebeneinander angeordnet werden.

Distanzelemente dringen radial typischerweise weniger weit am Amboss vor als Ambosssegmente. Zur Gewährleistung der Stabilität des Ambosses kann es vorteilhaft sein, wenn Distanzelemente dabei nur einen geringfügig kleineren Durchmesser aufweisen als Ambosssegmente.

Bevorzugt ist wenigstens eines der weiteren Elemente ein Schneidelement, insbesondere kann das Schneidelement zwischen zwei Ambosssegmenten angeordnet sein.

Durch Hinzufügen eines Schneidelementes kann ggf. ein zusätzlicher Bearbeitungsschritt bei der Herstellung eines Produkts zeit- und ortsgleich mit einem Ultraschallschweißvorgang realisiert werden. Beispielsweise kann überschüssiges Material abgeschnitten werden und/oder ein erzeugtes Produkt von weiteren erzeugten Produkten abgetrennt werden.

Weiter bevorzugt kann der Amboss wenigstens ein Prägeelement aufweisen. Insbesondere kann das Prägeelement zwischen zwei Ambosssegmenten angeordnet sein und vorzugsweise zum Einprägen von Verformungsbereichen in ein zu verschweißendes Material genutzt werden.

Verformungsbereiche weisen insbesondere eine vergleichsweise geringe Biegesteifigkeit auf. Alternativ oder zusätzlich können Einprägungen erzeugt werden, welche der ästhetischen Gestaltung und/oder haptischen Kenntlichmachung dienen.

Das Prägeelement kann ggf. zur Erzeugung von Einprägungen in nur einem der zu verschweißenden Werkstücke genutzt werden. Als Gegenlager für das Prägeelement kann insbesondere die Sonotrode genutzt werden. Alternativ kann ein neben der Sonotrode angeordnetes separates Gegenlager genutzt werden.

Falls die Sonotrode als Gegenlager für das Prägeelement genutzt wird, kann das Prägeelement ggf. ähnlich wie ein Ambosssegment eine Verpressung der Werkstücke erzeugen. Im vorliegenden Zusammenhang wird zur Abgrenzung zwischen Prägeelement und Ambosssegment davon ausgegangen, dass ein Ambosssegment ein stoffschlüssiges Verbinden zweier Materialien/Werkstücke hervorruft, während das Prägeelement zwar ggf. zwei übereinanderliegende Materialien/Werkstücke beeinflussen (prägen) kann, wobei allerdings keine stoffschlüssige Verbindung erzeugt wird.

Vorzugsweise kann der Amboss wenigstens ein Führungselement aufweisen. Insbesondere kann das Führungselement zwischen zwei Ambosssegmenten angeordnet sein.

Ein Führungselement kann insbesondere die Führung eines ggf. bandförmigen Werkstücks/Materials entlang einer Maschinenrichtung der entsprechenden Ultraschallschweißanlage unterstützen.

Falls das dem Amboss zugewandte Werkstück/Material vorstehende Elemente, wie beispielsweise Verschlusselemente eines Haftverschlussteils, aufweist, können diese mit dem Führungselement zum Erzeugen der Führungswirkung zusammenwirken. Ggf. können vorstehende Elemente durch das Führungselement elastisch verdrängt und in der Folge teilweise untergriffen werden. Ein Führungselement kann wahlweise auch an einem Längsrand eines Werkstücks/Materials angreifen.

Zusätzlich zu den beschriebenen weiteren Elementen, d. h. Distanz-, Schneid-, Präge- und Führungselementen, kann der Amboss zusätzliche weitere Elemente mit jeweils weiteren Funktionen aufweisen.

Vorzugsweise können Ambosssegmente und ggf. die weiteren Elemente miteinander verschraubt sein.

Beim Einsatz des erfindungsgemäßen Ambosses bei einem Ultraschallschweißvorgang sollte zwischen Ambosssegmenten (und ggf. weiteren Elementen) möglichst wenig, vorzugsweise kein, mechanisches Spiel vorhanden sein.

Vorzugsweise kann ein Spannelement, insbesondere eine Schraube, die Gesamtheit der Ambosssegmente (und ggf. weiterer Elemente) miteinander verspannen.

Das Spannelement ist vorzugsweise in radialer Richtung von der Drehachse beabstandet. Das Spannelement kann parallel zur Drehachse wirken. Mittels zueinander fluchtend aufgereihter Durchgangsausnehmungen in den Ambosssegmenten und ggf. den weiteren Elementen kann mithilfe des Spannelements auch eine Verdrehsicherung der jeweiligen Komponenten (Ambosssegmente bzw. weitere Elemente) zueinander erreicht werden bzw. eine Stellung der jeweiligen Komponenten zueinander vorgegeben werden. Das von dem Spannelement hervorgerufene Verspannen kann, wie oben erwähnt, von den Krägen unterstützt werden.

In Ausführungsformen können Ambosssegmente und die vorhandenen weiteren Elemente jeweils mehrere in ihrer Umfangsrichtung beabstandete Durchgangsausnehmungen zur Aufnahme eines Spannelementes aufweisen. Derart kann beim Aufbau des Ambosses ggf. durch Auswahl einer jeweiligen Durchgangsausnehmung beim Einführen des Spannelements eine von mehreren möglichen Drehstellungen für die jeweilige Komponente gewählt werden.

Insbesondere können mehrere Spannelemente an einem Amboss vorhanden sein.

Ambosssegmente und, sofern vorhanden, zwischen und/oder neben den Ambosssegmenten angeordnete weitere Elemente, können insbesondere formschlüssig aneinander ausgerichtet sein.

Ein jeweiliger Formschluss kann beispielsweise über Vorsprünge und entsprechende Ausnehmungen an den jeweiligen Flanken der scheibenförmigen Ambosssegmente bzw. der weiteren Elemente erreicht werden.

Alternativ oder zusätzlich können Ambosssegmente bzw. weitere Elemente mithilfe von Passstiften und Passbohrungen an ihren sich gegenseitig berührenden Flanken gekoppelt werden.

Vorzugsweise sind wenigstens zwei unterschiedliche Typen von Ambosssegmenten vorhanden, insbesondere mit unterschiedlichen effektiven Durchmessern.

Wenn zwei Ambosssegmente jeweils einen unterschiedlichen Durchmesser aufweisen, kann sich jeweils ein Schweißspalt unterschiedlicher Weite zur Sonotrode hin ergeben. Die Sonotrode weist typischerweise eine glatte (insbesondere in Achsrichtung des Ambosses nicht gestufte) Wirkfläche auf. Derart können insbesondere Schweißstellen bzw. Schweißlinien mit unterschiedlichen Eigenschaften erzeugt werden. Ein engerer Schweißspalt kann ggf. ein "intensiveres" Verschweißen bedingen und/oder die lokale Steifigkeit des Schweißverbunds verringern. Dadurch wird insbesondere ein höherer Integrationsgrad zwischen den verschweißten Materialien erreicht.

In Ausführungsformen können darüber hinaus unterschiedliche Durchmesser an einem Ambosssegment realisiert sein (d. h. ein Ambosssegment weist einen sich ändernden effektiven Durchmesser auf).

Vorzugsweise kann wenigstens eines der Ambosssegmente mehrere voneinander beabstandete Schweißvorsprünge aufweisen.

Mittels eines derart ausgestalteten Ambosssegments können bei der Verwendung des Ambosses an einer entsprechenden Ultraschallschweißanlage in Maschinenrichtung unterbrochene Schweißlinien erzeugt werden. Abhängig von der Ausdehnung eines jeweiligen Schweißvorsprungs bzw. der Distanz zwischen in Umfangsrichtung des Ambosssegments aufeinanderfolgenden Schweißvorsprüngen kann so ein bestimmtes Schweißmuster festgelegt sein. Das Schweißmuster kann insbesondere Einfluss auf die Steifigkeitsverteilung in einem erzeugten Produkt haben.

Insbesondere kann ein Amboss mehrere Ambosssegmente mit jeweiligen diskreten, in Umfangsrichtung beabstandeten Schweißvorsprüngen aufweisen. Die Ausdehnung der jeweiligen Schweißvorsprünge und Abstände zwischen Schweißvorsprüngen eines Ambosssegments können in Anpassung an ein bevorzugtes Schweißmuster entsprechend gewählt werden.

### Erfindungsgemäße Ultraschallschweißanlage

Die Aufgabe wird auch gelöst durch eine Ultraschallschweißanlage mit einer Sonotrode und einem zuvor beschriebenen modularen Amboss.

Die Ultraschallschweißanlage kann sich insbesondere zur Bearbeitung von bandförmigen Materialien/Werkstücken in einem kontinuierlichen Prozess eignen. Vorzugsweise werden die bandförmigen Materialien dabei entlang einer Maschinenrichtung bewegt. Alternativ oder zusätzlich können der Amboss und die Sonotrode (entgegen der Maschinenrichtung) bewegt werden.

Die Sonotrode kann insbesondere flach ausgebildet sein. Eines der zu verschweißenden Werkstücke gleitet im Rahmen eines kontinuierlichen Bearbeitungsprozesses insbesondere an der Sonotrode entlang, während der Amboss auf einem der zu verschweißenden Materialen abgerollt wird.

Alternativ kann die Sonotrode walzenförmig ausgebildet sein. Die walzenförmige Sonotrode ist typischerweise um eine zur Drehachse des Amboss parallele weitere Drehachse drehbar gelagert.

Ein drehbarer Amboss kann wahlweise frei drehbar an der Ultraschallschweißanlage angeordnet sein oder mittels einer Antriebseinheit rotiert werden.

Die Ultraschallschweißanlage eignet sich insbesondere zum Verschweißen eines Haftverschlussteils, das vorstehende Verschlusselemente aufweist, und eines Festlegteils. Das Festlegeteil kann insbesondere einen Vliesstoff aufweisen. Insofern betrifft die Erfindung auch die Verwendung der erfindungsgemäßen Ultraschallschweißanlage zum Verschweißen eines Haftverschlussteils, das vorstehende Verschlusselemente aufweist, und eines Festlegteils. Die Variationsmöglichkeiten bei der Konfiguration der erhaltenen Verschweißungen kommen dabei besonders vorteilhaft zur Geltung. Insbesondere können die Verschweißungen leicht für verschiedenartige Haftverschluss- und Festlegeteile angepasst werden, sei es im Rahmen einer Großserien- oder Kleinserienfertigung oder für Versuchszwecke zur Optimierung der Verschweißungen.

Bevorzugt weist/weisen ein derartiges Haftverschlussteil und/oder das Festlegeteil thermoplastisches Kunststoffmaterial, insbesondere Polyolefine, Polyester und/oder Polyamid, auf. Weiter bevorzugt weist das Haftverschlussteil bzw. das Festlegeteil biologisch abbaubares Kunststoffmaterial und/oder biobasiertes Kunststoffmaterial auf.

Über das Verschweißen des Haftverschlussteils mit dem Festlegeteil lässt sich beispielsweise ein Haftverschlusssystem für einen Hygieneartikel, insbesondere eine Windel, herstellen.

### Erfindungsgemäßer Bausatz

Die Aufgabe wird weiterhin gelöst durch einen Bausatz für einen modularen Amboss, aufweisend eine Vielzahl von Ambosssegmenten und vorzugsweise eine Vielzahl von weiteren Elementen, insbesondere wenigstens ein Distanzelement, wenigstens ein Schneidelement, wenigstens ein Prägeelement und/oder wenigstens ein Führungselement, wobei insbesondere durch Auswahl wenigstens zweier Ambosssegmente und ggf. wenigstens eines der weiteren Elemente ein zuvor beschriebener Amboss konfigurierbar ist.

Aus dem Bausatz kann auch ein Amboss mit nur einem Ambosssegment zusammengestellt werden. Neben dem einen Ambosssegment sind an dem Amboss dabei typischerweise weitere Elemente, insbesondere wenigstens ein Distanzelement, wenigstens ein Schneidelement, wenigstens ein Prägeelement und/oder wenigstens ein Führungselement, vorgesehen.

Der Bausatz ermöglicht es, durch Auswahl entsprechender Ambosssegmente und ggf. weiterer Elemente den Amboss verschiedenartig zu konfigurieren. Durch den Bausatz ist die Bereitstellung von unterschiedlich gestalteten Ambosstypen schnell und mit geringem Aufwand möglich. Es ist insbesondere auch denkbar, durch unterschiedliche Anordnung derselben Komponenten unterschiedliche Konfigurationen des Ambosses einzurichten.

Bevorzugt enthält der Bausatz jeweilige Ambosssegmente bzw. weitere Elemente mehrmals in gleicher Ausführung. Insbesondere ist der Bausatz durch Hinzufügen weiterer Ambosssegmente bzw. weiterer Elemente beliebig erweiterbar.

Ggf. kann der Bausatz neben Ambosssegmenten bzw. weiteren Elementen zusätzliche Teile, wie etwa Krägen, Wellen oder Spannelemente, enthalten.

Besonderes bevorzugt weist jedes der Ambosssegmente bzw. weiteren Elemente des Bausatzes eine eindeutige Bezeichnung, insbesondere in Form eines Codes, auf, woraus die genauen Spezifikationen der jeweiligen Komponente unmittelbar ablesbar sind (z. B. Durchmesser, Anzahl und Ausdehnung von Schweißvorsprüngen, axiale Ausdehnung, etc.). Derart kann der Aufbau, bzw. der gewünschte Aufbau, eines modularen Ambosses besonderes effektiv gegenüber Dritten kommuniziert werden oder in eine Datenverwaltung oder Simulation eingepflegt werden.

### Erfindungsgemäßes Verfahren

Die Aufgabe wird ferner gelöst durch ein Verfahren zum Festlegen der Gestaltung eines Ambosses für eine Ultraschallschweißanlage, wobei aus dem erwähnten Bausatz wenigstens zwei unterschiedliche zuvor beschriebene Ambosse (bzw. zwei verschiedene Konfigurationen eines Ambosses) zusammengestellt werden und mit diesen unterschiedlichen Ambossen (bzw. Konfigurationen) Probeverschweißungen durchgeführt werden, und wobei ein nichtmodularer Amboss für Serienverschweißungen entsprechend der Konfiguration eines der unterschiedlichen Ambosse (bzw. einer der Konfigurationen) hergestellt wird.

Derart lässt sich in rationeller Weise ein Amboss für eine Serienproduktion, insbesondere Großserienproduktion, gestalten. Insbesondere kann der Gestaltungsprozess in vorteilhafter Weise im Austausch mit Dritten (z. B. einem Hersteller von Ultraschallschweißanlagen, oder einem Abnehmer von verschweißten Werkstücken) erfolgen, da Experimente zügig und kostengünstig durchgeführt werden können.

Weitere Merkmale und Vorteile der Erfindung ergeben sich aus der Beschreibung, den Ansprüchen und der Zeichnung. Erfindungsgemäß können die vorstehend genannten und die noch weiter ausgeführten Merkmale jeweils einzeln für sich oder zu mehreren in beliebigen, zweckmäßigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Kurzbeschreibung der Zeichnung

Die Erfindung ist in der Zeichnung dargestellt und wird anhand von Ausführungsbeispielen beschrieben. Es zeigen:
- Fig. 1: eine Schnittansicht eines erfindungsgemäßen modularen Ambosses für eine erfindungsgemäße Ultraschallschweißanlage;
- Fig. 2a: eine perspektivische, ausschnittsweise Ansicht einer erfindungsgemäßen Ultraschallanlage mit einem weiteren erfindungsgemäßen modularen Amboss;
- Fign. 2b-2j: jeweils eine perspektivische Darstellung eines Zwischenschritts beim Aufbau des modularen Ambosses aus Fig. 2a;
- Fig. 3: eine perspektivische Darstellung eines weiteren erfindungsgemäßen modularen Ambosses;
- Fig. 4: eine geschnittene Seitenansicht eines Haftverschlusssystems, welches mit einem erfindungsgemäßen Amboss bzw. einer erfindungsgemäßen Ultraschallschweißanlage hergestellt wurde;
- Fig. 5: eine Draufsicht auf ein weiteres Haftverschlusssystem, welches mit einem erfindungsgemäßen Amboss bzw. einer erfindungsgemäßen Ultraschallschweißanlage hergestellt wurde;
- Fig. 6: eine perspektivische Darstellung einer weiteren erfindungsgemäßen Ultraschallschweißanlage mit einem erfindungsgemäßen modularen Amboss;
- Fig. 7: ein Flussdiagramm eines erfindungsgemäßen Verfahrens.

### Detaillierte Beschreibung der Erfindung und Zeichnung

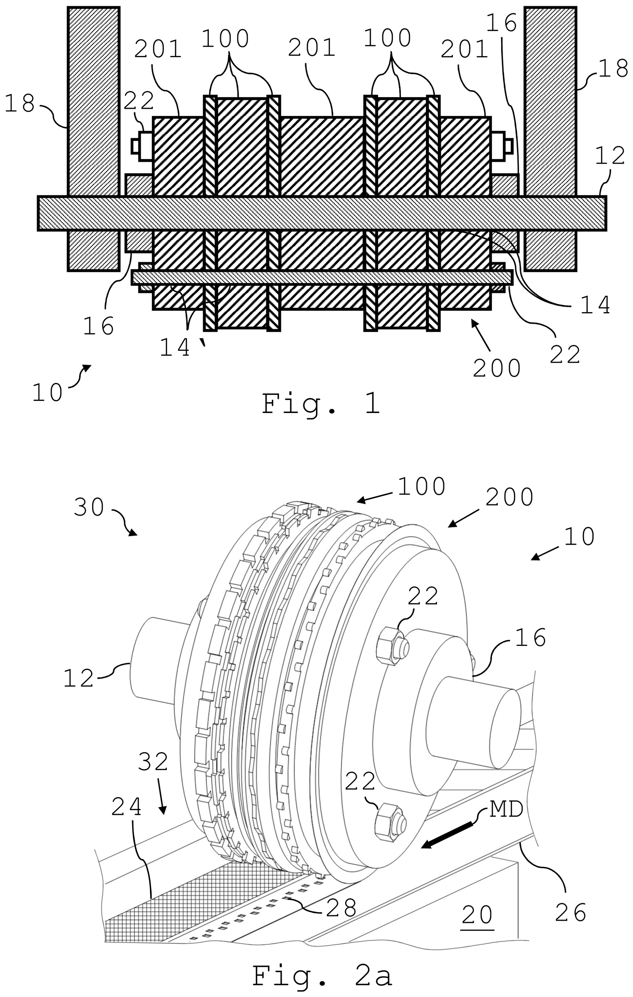

**Fig.** 1 zeigt eine entlang einer Drehachse **12** geschnittene Ansicht eines modularen Ambosses **10** für eine Ultraschallschweißanlage **30** (siehe Fig. 2a). Der Amboss 10 ist walzenförmig ausgebildet und weist eine Mehrzahl von scheibenförmigen Ambosssegmenten **100** sowie, vorzugsweise ebenfalls scheibenförmige, weitere Elementen **200** auf. Die Ambosssegmente 100 und die weiteren Elemente 200 sind auf der Drehachse 12 angeordnet, wobei die Drehachse 12 eine jeweilige zentrale Ausnehmung **14** der Ambosssegmente 100 und der weiteren Elemente 200 durchgreift.

Die Ambosssegmente 100 weisen insbesondere unterschiedliche effektive Durchmesser auf, wobei ein Ambosssegment 100 radial weiter vordringen kann als ein anderes Ambosssegment 100. Derart kann bei einem Ultraschallschweißvorgang ein Verschweißen mit unterschiedlichen Intensitäten aufgrund der unterschiedlichen Weite eines jeweiligen Schweißspaltes zwischen dem Amboss 10 bzw. dem jeweiligen Ambosssegment 100 und einer Sonotrode **20** (siehe Fig. 2a) erreicht werden. Dadurch können gezielt Eigenschaften einer jeweiligen Verschweißung beeinflusst werden.

Die weiteren Elemente 200 sind im dargestellten Ausführungsbeispiel als Distanzstücke **201** ausgebildet. Die Distanzstücke 201 können jeweils unterschiedliche Ausdehnungen insbesondere entlang der Drehachse 12 aufweisen. Distanzstücke 201 können insbesondere als separate Scheiben vorliegen und ggf. unmittelbar nebeneinander kombiniert werden, um eine gewünschte Ausdehnung (Distanz) zu erzielen. Alternativ oder zusätzlich können Distanzstücke ggf. an Ambosssegmenten 100 (als Vorsprung in Richtung der Drehachse 12) fest ausgebildet sein (nicht dargestellt).

Die Ambosssegmente 100 und die weiteren Elemente 200, hier Distanzstücke 201, sind in axialer Richtung mittels zweier Krägen **16** auf der Drehachse 12 fixiert. Zumindest einer der Krägen 16 kann von der Drehachse 12 zum Montieren der Ambosssegmente 100 bzw. der weiteren Elemente 200 entfernt werden. Der entfernbare Kragen 16 oder ggf. beide Krägen 16 kann/können beispielsweise mithilfe von radial zur Drehachse 12 vordringenden Stiftschrauben oder Stiften an der Drehachse 12 fixiert sein oder als Spannschelle(n) ausgebildet sein (nicht im Detail dargestellt).

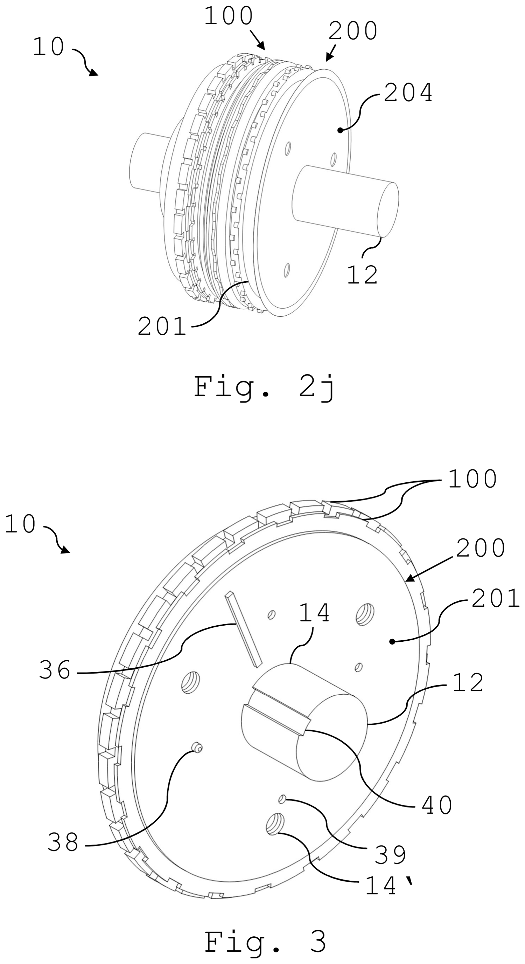

Die Drehachse 12 ist beidenends drehbar in einem jeweiligen Lagerblock **18** gelagert. Die Ambosssegmente 100 und/oder die weiteren Elemente 200 können insbesondere gegen ein Verdrehen gegenüber der Drehachse 12 gesichert sein. Dies kann beispielsweise über einen Formschluss erfolgen (vgl. Fig. 3) oder mittels weiterer Maßnahmen, wie beispielsweise einem Presssitz auf der Drehachse 12 oder axiales Verspannen gegen einen an der Drehachse 12 festgelegten Kragen 16, erreicht werden.

Im dargestellten Ausführungsbeispiel sind die Ambosssegmente 100 und die Distanzstücke 201 mittels mehrerer Spannelemente **22** miteinander verspannt. Eines der Spannelemente 22 ist geschnitten dargestellt. Die Spannelemente 22 durchgreifen dabei zueinander fluchtend aufgereihte Ausnehmungen **14'** in den jeweiligen Ambosssegmenten 100 bzw. weiteren Elementen 200. Die Spannelemente 22 sind im dargestellten Ausführungsbeispiel als Gewindestangen mit schraubbaren Muttern an beiden Seiten ausgebildet. Gleichermaßen kann ein Spannelement 22 als Schraube, insbesondere als Passschraube, ausgebildet sein, insbesondere wobei ein Schaft der Passschraube in den Ausnehmungen 14' spielfrei ist. Insbesondere kann ein als Schraube ausgebildetes Spannelement 22 beispielsweise in ein äußeres, dem Schraubenkopf gegenüberliegendes, Distanzstück 201 oder einen auf der Drehachse 12 befestigten Kragen 16 mit eingebrachtem Gewinde eingeschraubt werden (nicht näher dargestellt). Spannelemente 22 können ggf. mit zusätzlichen Sicherungselementen gegen ein unbeabsichtigtes Lösen gesichert sein (nicht dargestellt).

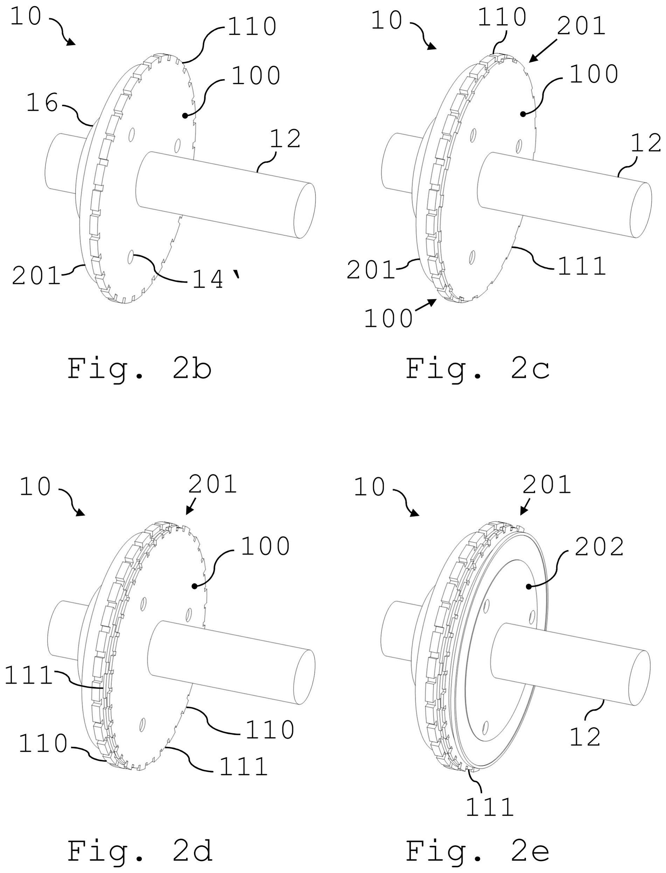

**Fig. 2a** zeigt eine perspektivische Darstellung einer Ultraschallschweißanlage 30 mit einer weiteren Ausführungsvariante eines modularen Ambosses 10 mit einer Mehrzahl von Ambosssegmenten 100 und weiteren Elementen 200. Der Aufbau des Ambosses 10 sowie die jeweilige Funktion der Ambosssegmente 100 und der weiteren Elemente 200 wird in der Beschreibung zu den Fign. 2b-2j genauer erläutert. (In den Figuren der Zeichnung sind mehrfach auftretende Elemente zur besseren Übersichtlichkeit i. d. R. nur zum Teil mit Bezugszeichen versehen.)

Die Sonotrode 20, welche mit dem Amboss 10 zum Erzeugen einer jeweiligen Verschweißung zusammenwirkt, ist ausschnittsweise abgebildet. Die Sonotrode 20 kann insbesondere, wie dargestellt, flach ausgebildet sein. Alternativ kann sie analog zum Amboss 10 walzenförmig ausgebildet sein.

Im dargestellten Ausführungsbeispiel wird eine Bahn eines Haftverschlussteils **24** mit einer Bahn eines Festlegeteils **26** entlang einer Maschinenrichtung **MD** der Ultraschallschweißanlage 30 verschweißt. Zeitgleich werden Einprägungen **28** in das Festlegeteil 26 eingebracht und das Festlegeteil 26 bzw. der erzeugte Verbund aus Festlegeteil 26 und Haftverschlussteil 24 wird zugeschnitten. Vorzugsweise wird das Haftverschlussteil 24 gemeinsam mit dem Festlegeteil 26 relativ zur Ultraschallschweißanlage 30 bewegt, wobei der Amboss 10 auf der jeweiligen ihm zugewandten Oberfläche abrollt. Das Festlegeteil 26 kann dabei insbesondere an der Sonotrode 20 entlanggleiten.

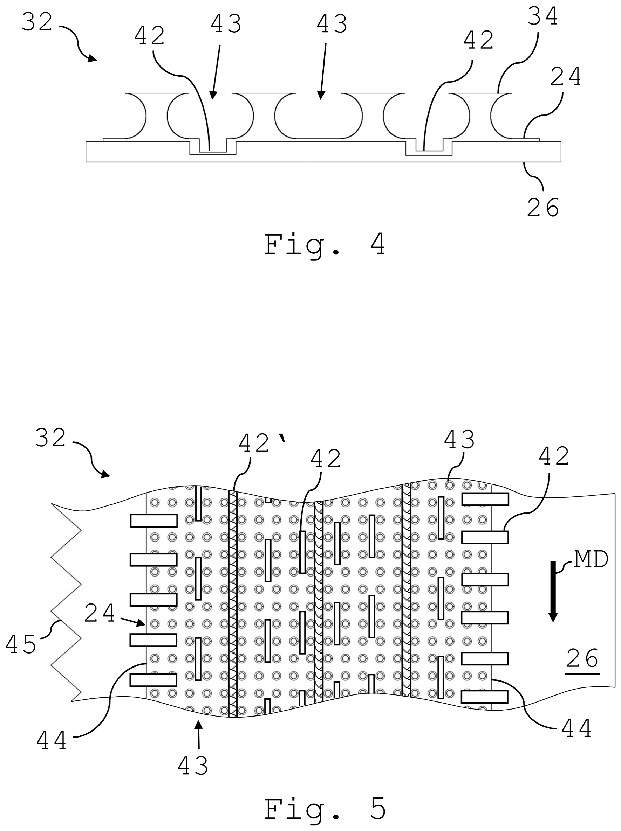

Dieserart kann beispielsweise eine rationelle Fertigung eines Haftverschlusssystems **32** erfolgen, bei dem ein Haftverschlussteil 24 und ein Festlegeteil 26 bereichsweise miteinander verschweißt sind. Das Haftverschlussteil 24 kann dabei insbesondere auf seiner Vorderseite vorstehende Verschlusselemente **34** (siehe Fig. 4) aufweisen. Ggf. können Ambosssegmente 100 und/oder weitere Elemente 200 zwischen vorstehende Verschlusselemente 34 eingreifen, ohne dabei unmittelbar benachbarte Verschlusselemente 34 zu beschädigen. Erzeugte Verschweißungen sind in Fig. 2a nicht im Detail dargestellt (vgl. dazu Fign. 4 und 5).

Der modulare Amboss 10 weist analog zu dem Amboss 10 aus Fig. 1 eine als mechanische Welle ausgebildete Drehachse 12 auf. Die verschiedenen Ambosssegmente 100 und weiteren Elemente 200 sind auf der Drehachse 12 angeordnet und in Achsrichtung mithilfe von Krägen 16 fixiert. Die Drehachse 12 ist insbesondere beidseitig an Lagerblöcken 18 (siehe Fig. 1) gelagert. Die Lagerblöcke 18 sind in Fig. 2a zur besseren Übersichtlichkeit ausgeblendet. Spannelemente 22 verspannen die einzelnen Ambosssegmente 100 und weiteren Elemente 200 miteinander.

Der Aufbau des in Fig. 2a dargestellten Ambosses 10 ist beispielhaft zu verstehen. Es soll insbesondere eine große Anzahl unterschiedlicher Ambosssegmente 100 und weiterer Elemente 200 zur Erläuterung von deren jeweiliger Funktion dargestellt werden. Gleichermaßen sind die dargestellten Komponenten nicht als abschließende Auflistung möglicher Komponenten zu betrachten. Ggf. kann ein jeweiliger modularer Amboss 10 zusätzliche Ambosssegmente 100 und/oder weitere Elemente 200 aufweisen. Insbesondere können bestimmte Ambosssegmente 100 und/oder weitere Elemente 200 mehrfach auftreten. Unter Umständen kann eine bezüglich einer Ebene senkrecht zur Drehachse 12 spiegelsymmetrische Anordnung von Ambosssegmenten 100 und/oder weiteren Elementen 200 bevorzugt werden (vgl. Fig. 1).

Die Fign. 2b-2j zeigen sukzessive den Zusammenbau des Ambosses 10 aus Fig. 2a. Die in den Fign. 2b-2j dargestellten Konfigurationen können alternativ jeweils als alleinstehendes Ausführungsbeispiel eines jeweiligen weiteren modularen Ambosses 10 betrachtet werden. **Fig. 2b** zeigt die Drehachse 12 mit einem der Krägen 16, einem Distanzstück 201 sowie einem Ambosssegment 100. Das Ambosssegment 100 weist diskrete, in Umfangsrichtung voneinander beabstandete Schweißvorsprünge **110** auf. Mittels der Schweißvorsprünge 110 kann bei einem Abrollen des drehbar gelagerten Ambosses 10 eine unterbrochene Schweißlinie erzeugt werden. Am Ambosssegment 100 sind die Ausnehmungen 14' zur Aufnahme der Spannelemente 22 (siehe Fig. 2a) erkennbar.

**Fig. 2c** zeigt den sich im Aufbau befindenden Amboss 10 aus den Fign. 2a und 2b. Im Vergleich zu Fig. 2b wurde hier zunächst ein weiteres Distanzstück 201 auf die Drehachse 12 aufgeschoben (verdeckt) und anschließend ein Ambosssegment 100 hinzugefügt. Das hinzugekommene Ambosssegment 100 weist ebenfalls voneinander beabstandete Schweißvorsprünge **111** auf. Die Schweißvorsprünge 111 dringen hier radial weniger weit vor als die Schweißvorsprünge 110 des vorhergehenden Ambosssegments 100. Die Schweißvorsprünge 111 sind in axialer Richtung des Ambosses 10 gesehen gegenüber den Schweißvorsprüngen 110 insbesondere zumindest teilweise versetzt. Das hinzugekommene Ambosssegment 100 ist deutlich schmäler als das vorhergehende Ambosssegment 100. Dadurch kann es sich ggf. insbesondere zum Eingriff zwischen vorstehende Verschlusselemente 34 (siehe Fig. 4) eines zur verschweißenden Haftverschlussteils 24 (siehe Fig. 2a) eignen. Insbesondere können Verschlusselemente 34, welche sich bei einem Schweißvorgang zwischen den Ambosssegmenten 100 (d. h. unterhalb des Distanzstücks 201) befinden, unbeeinträchtigt bleiben. Das vorhergehende Ambosssegment 100 kann sich insbesondere zum Verschweißen eines Längsrandes **44** (siehe Fig. 5) des Haftverschlussteils 24 eignen.

In **Fig. 2d** ist gegenüber Fig. 2c ein weiteres Distanzstück 201 (verdeckt) sowie ein weiteres Ambosssegment 100 hinzugekommen. Das hinzugekommene Ambosssegment 100 weist hier, insbesondere in Umfangsrichtung alternierend, voneinander beabstandete erste Schweißvorsprünge 110 und weniger weit vordringende zweite Schweißvorsprünge 111 auf. Schweißvorsprünge 110 bzw. 111 können insbesondere jeweils unterschiedliche Ausdehnungen entlang des Umfangs des jeweiligen Ambosssegments 100 aufweisen. In Ausführungsformen können jeweilige Schweißvorsprünge 110, 111 insbesondere eine Vielzahl (mehr als zwei) verschiedener Höhen aufweisen.

In **Fig. 2e** wurde wiederum ein Distanzstück 201 aufgeschoben. Anschließend wurde ein Führungselement **202** auf die Drehachse 12 aufgeschoben. Das Führungselement 202 ist bei einem Schweißvorgang insbesondere nicht mit seinem radialen Außenrand in Kontakt mit dem zu verschweißenden Material. Es dringt radial somit weniger weit vor als die Schweißvorsprünge 111.

Demgegenüber kann sich das Führungselement 202 insbesondere zum teilweisen Eingriff zwischen reihenförmig angeordnete Verschlusselemente 34 (siehe Fig. 4) eignen, wobei eine Führung eines Haftverschlussteils 24 (siehe Fig. 2a) entlang der Maschinenrichtung MD (siehe Fig. 2a) realisiert werden kann.

Im dargestellten Ausführungsbeispiel eignet sich das Führungselement 202 insbesondere zum teilweisen Untergreifen von Verschlusselementen 34, welche einen Überhang aufweisen (die beispielsweise pilzförmig, palmenförmig und/oder hakenförmig sind). Beim Einführen des Führungselements 202 zwischen die Verschlusselemente 34 kann insbesondere ein geringfügiges elastisches Verdrängen derselben erfolgen, um das Untergreifen zu ermöglichen. In Ausführungsformen kann auch zumindest ein Ambosssegment 100 für ein derartiges Untergreifen ausgestaltet sein (nicht dargestellt).

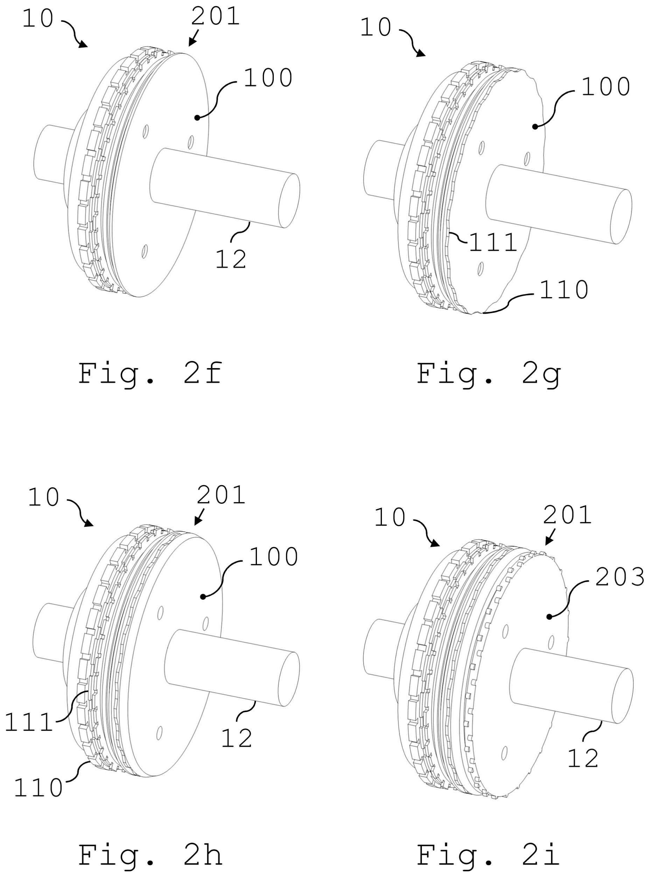

In **Fig. 2f** wurde ein weiteres Distanzstück 201 und ein weiteres Ambosssegment 100 auf die Drehachse 12 aufgeschoben. Das hinzugekommene Ambosssegment 100 eignet sich dabei insbesondere zur Erzeugung einer durchgehenden Schweißlinie.

Ein in **Fig. 2g** neu hinzugekommenes Ambosssegment 100 weist wiederum Schweißvorsprünge 110, 111 unterschiedlicher Höhe auf. Hierbei sind die Schweißvorsprünge 110, 111 in Umfangsrichtung nicht voneinander beabstandet, sondern gehen jeweils rampenförmig ineinander über. Bei einem Schweißvorgang entsteht so eine durchgehende Schweißlinie, wobei die Verschweißung bereichsweise abwechselnd unterschiedliche Eigenschaften aufweist.

In der zuvor erläuterten Weise wurde in **Fig. 2h** wieder ein Distanzstück 201 auf die Drehachse 12 aufgeschoben und anschließend ein weiteres Ambosssegment 100 angeordnet.

Das hinzugekommene Ambosssegment 100 weist in axialer Richtung der Drehachse 12 insbesondere einen sich verkleinernden (bzw. vergrößernden) Durchmesser auf (es kann eine Kegelfläche oder eine gewölbte Konusfläche aufweisen). Dabei kann eine Schweißlinie erzeugt werden, welche in ihrer Querrichtung variierende Eigenschaften aufweist. Insbesondere kann der Durchmesser des hinzugekommenen Ambosssegments 100 beispielsweise von einem den ersten Schweißvorsprüngen 110 entsprechenden Durchmesser auf einen den zweiten Schweißvorsprüngen 111 entsprechenden Durchmesser abnehmen.

**Fig. 2i** zeigt ein nach einem weiteren Distanzstück 201 auf der Drehachse 12 angeordnetes Prägeelement **203.** Das Prägeelement 203 eignet sich insbesondere zum Herstellen ästhetisch ansprechender Einprägungen 28 (siehe Fig. 2a). Zusätzlich oder alternativ können Einprägungen 28 eine funktionale (verhaltensmodifizierende) Aufgabe an dem erzeugten Produkt erfüllen. Beispielsweise können sie zur Ausbildung von Verformungsbereichen in einem Festlegeteil 26 (siehe Fig. 2a) dienen.

Im Rahmen einer Ultraschallschweißanlage 30 kann das Prägeelement 203 insbesondere die Sonotrode 20 als Gegenlager nutzen (wie dies in Fig. 2a dargestellt ist). Ggf. kann für das Prägeelement 203 alternativ ein separates Gegenlager (ohne Sonotrodenfunktionalität) vorgesehen sein (nicht dargestellt).

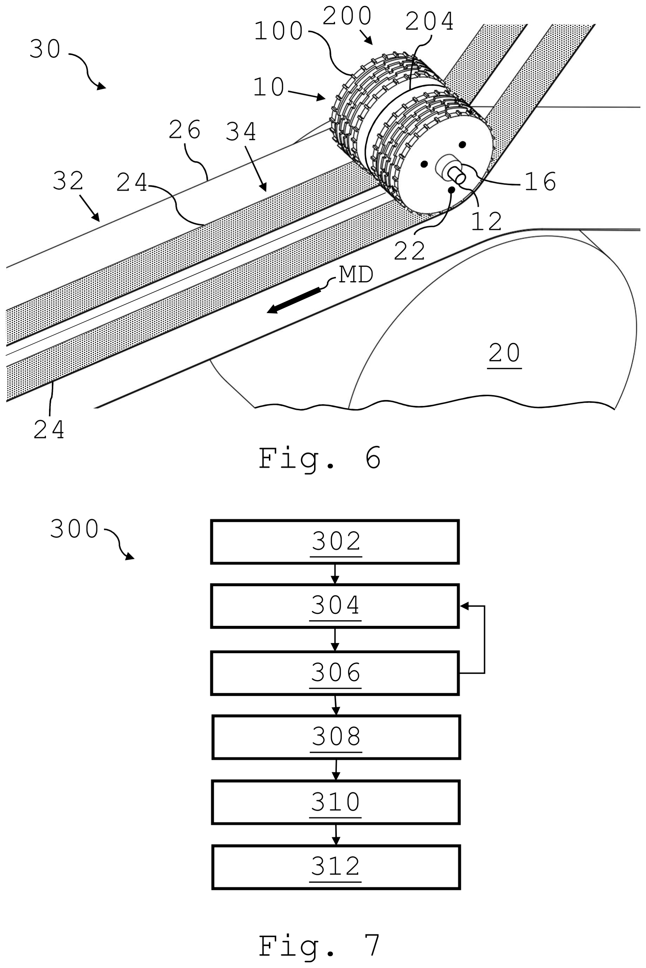

In **Fig. 2j** wurden ein Distanzstück 201 und ein Schneidelement **204** zum Amboss 10 hinzugefügt. Mittels des Schneidelementes 204 kann ein erzeugtes Produkt während eine Schweißvorganges zeitgleich zugeschnitten werden (zumindest entlang der Maschinenrichtung MD; siehe Fig. 2a). Insbesondere können so beispielsweise mehrere in einem Arbeitsgang produzierte Haftverschlusssysteme 32 (siehe Fig. 2a) voneinander getrennt (vgl. Fig. 6) oder zugeschnitten werden.

Das dargestellte Schneidelement 204 ist als mit dem Amboss 10 rotierendes Scheibenmesser ausgebildet. Alternativ kann das Schneidelement 204 beispielsweise eine in Umfangsrichtung wellenförmige Schneide zur Erzeugung einer S-förmigen oder zickzackförmigen Schnittlinie oder beabstandete Schneidvorsprünge zur Erzeugung einer Perforationslinie aufweisen (nicht dargestellt).

Das Schneidelement 204 kann, analog zum Prägeelement 203, die Sonotrode 20 als Gegenlager nutzen oder mit einem separaten Gegenlager zusammenwirken.

Um zum in Fig. 2a dargestellten Amboss 10 zu gelangen, wird ein weiteres Distanzstück 201 und ein weiterer Kragen 16 auf der Drehachse 12 angeordnet sowie die Verspannung mittels der Spannelemente 22 vorgenommen.

Der Amboss 10 kann unter mehrmaligen erneuten Anordnen der Ambosssegmente 100 und/oder der weiteren Elemente 200 in verschiedenen Weisen konfiguriert werden. Insbesondere können dabei zusätzliche, hier nicht dargestellte, Ambosssegmente 100 und/oder weitere Elemente 200 zum Einsatz kommen. Bevorzugt können bestimmte Ambosssegmente 100 und/oder weitere Elemente 200 jeweils mehrmals an einem Amboss 10 vorkommen. Mit einem jeweiligen Amboss 10 können insbesondere Probeverschweißungen vorgenommen werden bzw. Kleinserien produziert werden. Vorzugsweise kann ein vorteilhaft gestalteter Ambosses 10 als Prototyp bzw. Muster für einen weniger modularen, ggf. aus einem Stück hergestellten, Großserienamboss dienen.

**Fig. 3** zeigt eine perspektivische Ansicht einer weiteren Ausführungsform eines Ambosses 10. Der Amboss 10 weist zwei Ambosssegmente 100 auf. Ein Distanzstück 201, welches insbesondere mit einem nachfolgend anzuordnenden Abosssegment 100 oder weiteren Element 200 (vgl. Fign. 2a-2j) in Anlage geraten soll, weist hier einen Vorsprung **36** auf. Der Vorsprung 36 kann sich insbesondere zum Formschluss mit einer entsprechend ausgebildeten Ausnehmung in einem ggf. nachfolgend anzuordnenden Ambosssegment 100 oder weiteren Element 200 eignen (nicht dargestellt). Derart kann beispielsweise zusätzlich zu der Fixierung durch Spannelemente 22 an den Ausnehmungen 14' ein Verdrehen der beiden formschlüssig verbundenen Komponenten gegeneinander unterbunden werden bzw. eine relative Anordnung der Komponenten zueinander vorgegeben werden. Die Ausnehmungen 14' können dadurch insbesondere größere Toleranzbereiche aufweisen, da die Spannelemente 22 ausschließlich zum Verspannen (nicht zum Positionieren) genutzt werden können. In Ausführungsformen können Ausnehmungen 14' ggf. als gebogene Langlöcher ausgebildet sein (nicht dargestellt). Insbesondere kann ein jeweiliges Ambosssegment 100 oder weiteres Element 200 mehrere Vorsprünge 36 und/oder Ausnehmungen aufweisen (nicht dargestellt). Die Vorsprünge 36 und Ausnehmungen können so angeordnet sein, dass mehrere unterschiedliche Drehstellungen zweier benachbarter Komponenten 100/200 relativ zueinander eingerichtet werden können.

Alternativ, oder ggf. zusätzlich, können zu diesem Zweck Passstifte **38** vorgesehen sein. Zum Zusammenwirken mit den Passstiften 28 können Passbohrungen **39** in einer jeweiligen Komponente vorgesehen sein (hier beispielhaft an dem Distanzstück 201 dargestellt).

Weiterhin kann die Drehachse 12 alternativ oder zusätzlich für einen Formschluss vorgesehene Konstruktionsmerkmale aufweisen. Im dargestellten Ausführungsbeispiel weist die Drehachse 12 beispielsweise eine Nut **40** in Achsrichtung auf. Mittels einer entsprechenden Ausgestaltung der zentralen Ausnehmung 14 der Ambosssegmente 100 und weiteren Elemente 200 können die jeweiligen Komponenten gegenüber einem Verdrehen auf der Drehachse 12 gesichert werden. Ggf. kann die Drehachse 12 mehrere Nuten 40 aufweisen (nicht dargestellt).

**Fig. 4** zeigt ein Haftverschlusssystem 32 in einer Schnittansicht. Zur besseren Übersichtlichkeit sind die Schnittflächen nicht schraffiert dargestellt. Ein Haftverschlussteil 24 ist zur Ausbildung des Haftverschlusssystems 32 an Schweißstellen **42** mit einem Festlegeteil 26 verschweißt. Das Haftverschlussteil 24 weist vorstehende Verschlusselemente 34 auf.

Die Verschlusselemente 34 sind im dargestellten Beispiel palmenförmig ausgebildet und eignen sich insbesondere zum Zusammenwirken (Herstellen einer lösbaren Haftverbindung) mit einem Gewebe oder Vlies. Ggf. kann auch eine Haftverbindung zwischen den Verschlusselementen 34 und dem Festlegeteil 26 ermöglicht sein. Das Haftverschlusssystem 32 kann zu diesem Zweck beispielsweise zusammengeklappt oder als Ring geschlossen werden.

Das dargestellte Haftverschlusssystem 32 kann insbesondere mit einem erfindungsgemäßen Amboss 10 bzw. mit einer erfindungsgemäßen Ultraschallschweißanlage 30 (siehe Fig. 2a) hergestellt worden sein. Ambosssegmente 100 haben bei der Erzeugung der Schweißstellen 42 dabei in Zwischenräume **43** zwischen benachbarte Verschlusselemente 34 eingegriffen. Beim Schweißvorgang wurden keine Verschlusselemente 34 beeinträchtigt oder zerstört. Insbesondere konnte über die erwähnte Modularität des Ambosses 10 der Abstand sowie die Breite der verwendeten Ambosssegmente 100 genau an die Geometrie des Haftverschlussteils 24 angepasst werden.

**Fig. 5** zeigt ausschnittsweise ein weiteres Haftverschlusssystem 32, welches mit einem erfindungsgemäßen Amboss 10 bzw. der entsprechenden Ultraschallschweißanlage 30 (siehe Fig. 2a) hergestellt wurde, in einer Draufsicht.

Schweißstellen 42 erstrecken sich bereichsweise entlang der Maschinenrichtung MD in Zwischenräumen 43 zwischen Verschlusselementen 34 bzw. an den Längsrändern 44 eines Haftverschlussteils 24.

Schweißstellen 42 an den Längsrändern 44 können sich insbesondere bis über den jeweiligen Längsrand 44 hinaus auf ein Festlegeteil 26 erstrecken. Dabei gibt es am über den Längsrand 44 hinausreichenden Teil der entsprechenden Schweißstelle 42 keine Verbindung zwischen Haftverschlussteil 24 und Festlegeteil 26, sondern lediglich eine "Schweißung" innerhalb des Festlegeteils 26.

Insbesondere abhängig von der Höhe der jeweiligen Schweißvorsprünge 110, 111 (siehe Fig. 2c) bzw. vom effektiven Durchmesser eines jeweiligen Ambosssegments 100 können sich Schweißstellen 42 mit unterschiedlichen Eigenschaften bzw. unterschiedlicher Ausbildung ergeben. Das in Fig. 5 dargestellte Haftverschlusssystem 32 weist beispielsweise durchgehende Schweißlinien **42'** auf, welche insbesondere über einen geringeren Schweißspalt erzeugt wurden als die anderen, hier zusätzlich voneinander beabstandeten Schweißstellen 42.

Der gezackte Außenrand **45** des Haftverschlusssystems kann insbesondere mittels eines Schneidelements 204 (siehe Fig. 2j) erzeugt worden sein.

**Fig. 6** zeigt eine weitere Ultraschallschweißanlage 30, wobei ein erfindungsgemäßer Amboss 10 mit Ambosssegmenten 100 und weiteren Elementen 200 zur kontinuierlichen Herstellung von Haftverschlusssystemen 32 im Rahmen einer Parallelbearbeitung eingesetzt wird.

In Fig. 6 ist eine Drehachse 12 sowie ein Kragen 16 des Ambosses 10 sichtbar. Lagerblöcke 18 (siehe Fig. 1) sind zu besseren Übersichtlichkeit ausgeblendet. Spannelemente 22 sind lediglich angedeutet.

Der rotierende, walzenförmige Amboss 10 und eine rotierende, walzenförmige Sonotrode 20 wirken zur Herstellung von jeweiligen Schweißstellen 42 und/oder Schweißlinien 42' (siehe Fig. 5) zusammen. Fig. 6 zeigt ein bandförmiges Festlegeteil 26 sowie zwei bandförmige Haftverschlussteile 24, welche in einer Maschinenrichtung MD zusammengeführt und miteinander verschweißt werden.

Die beiden Haftverschlusssysteme 32 werden bei dem Ultraschallschweißprozess mittels des Schneidelements 204 parallel zur Maschinenrichtung MD voneinander getrennt.

**Fig. 7** zeigt ein Flussdiagramm eines erfindungsgemäßen Verfahrens **300.** Schritt **302** umfasst das Bereitstellen eines oben beschriebenen Bausatzes. In Schritt **304** wird ein modularer Amboss 10 mit zumindest einer Komponente des Bausatzes angefertigt. Schritt **306** umfasst das Schweißen mit dem zuvor konfigurierten Amboss 10. Die Schritte 304 und 306 werden in der Folge zumindest einmal wiederholt, wobei der Amboss 10 jeweils in einer anderen Konfiguration (bzw. ein weiterer Amboss 10) zusammengestellt und zum Erzeugen einer Verschweißung genutzt wird. In Schritt **308** wird eine der zuvor getesteten Konfigurationen (bzw. einer der getesteten Ambosse 10) ausgewählt. Anschließend wird in Schritt **310** ein nichtmodularer Amboss auf Basis des gewählten modularen Ambosses 10 hergestellt und in Schritt **312** für Serienverschweißungen genutzt.

Unter Vornahme einer Zusammenschau der Figuren der Zeichnung betrifft die Erfindung einen modularen Amboss 10 für eine Ultraschallschweißanlage 30, welcher insbesondere aus einem Bausatz aus einer Mehrzahl von Ambosssegmenten 100 und ggf. weiteren Elementen 200 konfiguriert werden kann. Ambosssegmente 100 dienen im Zusammenwirken mit einer Sonotrode 20 zur Erzeugung von Schweißstellen 42 bzw. Schweißlinien 42'. Als weitere Elemente 200 kommen beispielsweise Distanzelemente 201, Führungselemente 202, Prägeelemente 203 oder Schneidelemente 204 in Frage. Ambosssegmente 100 und ggf. weitere Elementen 200 sind lösbar aneinander befestigt, sodass der Amboss 10 mehrfach neu konfiguriert werden kann.

Die Erfindung betrifft auch ein Verfahren 300, wobei ein erfindungsgemäßer Amboss 10 in wenigstens zwei Konfigurationen, oder anders ausgedrückt wenigstens zwei erfindungsgemäße Ambosse 10, die aus demselben Bausatz erhalten sein können, für Probeverschweißungen genutzt wird/werden. Auf Basis der Probeverschweißungen wird anschließend eine vorteilhafte Konfiguration identifiziert und ein entsprechender nichtmodularer (d. h. weniger oder nicht segmentierter) Amboss auf Basis einer der zuvor verwendeten Konfigurationen des modularen Ambosses 10 hergestellt und für Serienverschweißungen genutzt.

### Bezugszeichenliste

| | | | |
|---|---|---|---|
| 10 | Amboss | 45 | Außenrand |
| 12 | Drehachse | | |
| 14 | zentrale Ausnehmung | 100 | Ambosssegment |
| 14' | Ausnehmung | 110 | Schweißvorsprung |
| 16 | Kragen | 111 | Schweißvorsprung |
| 18 | Lagerblock | 200 | weiteres Element |
| 20 | Sonotrode | 201 | Distanzelement |
| 22 | Spannelement | 202 | Führungselement |
| 24 | Haftverschlussteil | 203 | Prägeelement |
| 26 | Festlegeteil | 204 | Schneidelement |
| 28 | Einprägung | | |
| 30 | Ultraschallschweißanlage | 300 | Verfahren |
| 32 | Haftverschlusssystem | 302 | Bereitstellen des Bausatzes |
| 34 | Verschlusselement | 304 | Amboss konfigurieren |
| 36 | Vorsprung | 306 | Schweißen |
| 38 | Passstift | 308 | Auswahl einer Konfiguration |
| 39 | Passbohrung | 310 | Serienamboss herstellen |
| 40 | Nut | 312 | Serienverschweißung durchführen |
| 42 | Schweißstelle | | |
| 42' | Schweißlinie | | |
| 43 | Zwischenraum | MD | Maschinenrichtung |
| 44 | Längsrand | | |

## Patentansprüche

1. Modularer Amboss (10) für eine Ultraschallschweißanlage (30), aufweisend eine Mehrzahl von Ambosssegmenten (100) zum Zusammenwirken mit einer Sonotrode (20), wobei die Ambosssegmente (100) lösbar aneinander befestigt sind.

2. Amboss (10) nach Anspruch 1, wobei der Amboss (10) eine Drehachse (12) aufweist.

3. Amboss (10) nach Anspruch 2, wobei die Drehachse (12) eine zentrale Ausnehmung (14) der Ambosssegmente (100) durchgreift.

4. Amboss (10) nach einem der Ansprüche 2 oder 3, wobei die Ambosssegmente (100) und, sofern vorhanden, zwischen und/oder neben den Ambosssegmenten (100) angeordnete weitere Elemente (200, 201, 202, 203, 204), in axialer Richtung auf der Drehachse (12) fixiert sind, insbesondere durch zumindest einen lösbaren Kragen (16).

5. Amboss (10) nach einem der Ansprüche 2 bis 4, wobei die Drehachse (12) beidseits drehbar in einem Lagerblock (18) gelagert ist.

6. Amboss (10) nach einem der vorhergehenden Ansprüche, weiterhin aufweisend wenigstens ein Distanzelement (201), insbesondere wobei das Distanzelement (201) zwischen zwei Ambosssegmenten (100) angeordnet ist.

7. Amboss (10) nach einem der vorhergehenden Ansprüche, weiterhin aufweisend wenigstens ein Schneidelement (204), insbesondere wobei das Schneidelement (204) zwischen zwei Ambosssegmenten (100) angeordnet ist.

8. Amboss (10) nach einem der vorhergehenden Ansprüche, weiterhin aufweisend wenigstens ein Prägeelement (203), insbesondere wobei das Prägeelement (203) zwischen zwei Ambosssegmenten (100) angeordnet ist, vorzugsweise zum Einprägen von Verformungsbereichen in ein zu verschweißendes Material.

9. Amboss (10) nach einem der vorhergehenden Ansprüche, weiterhin aufweisend wenigstens ein Führungselement (202), insbesondere wobei das Führungselement (202) zwischen zwei Ambosssegmenten (100) angeordnet ist.

10. Amboss (10) nach einem der vorhergehenden Ansprüche, wobei die Ambosssegmente (100) miteinander verschraubt sind.

11. Amboss (10) nach einem der vorhergehenden Ansprüche, wobei ein Spannelement (22), insbesondere eine Schraube, die Gesamtheit der Ambosssegmente (100) miteinander verspannt.

12. Amboss (10) nach einem der vorhergehenden Ansprüche, wobei die Ambosssegmente (100) und, sofern vorhanden, zwischen und/oder neben den Ambosssegmenten (100) angeordnete weitere Elemente (200, 201, 202, 203, 204), formschlüssig aneinander ausgerichtet sind.

13. Amboss (10) nach einem der vorhergehenden Ansprüche, wobei wenigstens zwei unterschiedliche Typen von Ambosssegmenten (100) vorhanden sind, insbesondere mit unterschiedlichen effektiven Durchmessern.

14. Amboss (10) nach einem der vorhergehenden Ansprüche, wobei wenigstens eines der Ambosssegmente (100) mehrere voneinander beabstandete Schweißvorsprünge (110, 111) aufweist.

15. Ultraschallschweißanlage (30) mit einer Sonotrode (20) und einem Amboss (10) nach einem der Ansprüche 1 bis 14.

16. Verwendung einer Ultraschallschweißanlage (30) nach Anspruch 15 zum Verschweißen eines Haftverschlussteils (24), das vorstehende Verschlusselemente (34) aufweist, und eines Festlegteils (26), insbesondere aus einem Vliesstoff.

17. Bausatz für einen Amboss (10), aufweisend eine Vielzahl von Ambosssegmenten (100) und vorzugsweise eine Vielzahl von weiteren Elementen (200, 201, 202, 203, 204), insbesondere wenigstens ein Distanzelement (201), wenigstens ein Schneidelement (204), wenigstens ein Prägeelement (203) und/oder wenigstens ein Führungselement (202), wobei insbesondere durch Auswahl wenigstens zweier Ambosssegmente (100) ein Amboss nach einem der Ansprüche 1 bis 14 konfigurierbar ist.

18. Verfahren (300) zum Festlegen der Gestaltung eines Ambosses (10) für eine Ultraschallschweißanlage (30), wobei aus dem Bausatz nach Anspruch 15 wenigstens zwei unterschiedliche Ambosse (10), insbesondere Ambosse (10) nach einem der Ansprüche 1 bis 14, zusammengestellt werden und mit diesen unterschiedlichen Ambossen (10) Probeverschweißungen durchgeführt werden, und wobei ein nichtmodularer Amboss für Serienverschweißungen entsprechend der Konfiguration eines der unterschiedlichen Ambosse (10) hergestellt wird.
